# EUROPEAN PATENT APPLICATION

(11) **EP 1 231 470 A1**
(43) Date of publication of application: **14.08.2002**
(21) Application number: 00971755.4
(22) Date of filing: 02.11.2000
(51) Int. Cl.: G01N 33/543, G01N 33/547

(54) **METHOD FOR IMMOBILIZING SUBSTANCES**

(30) Priority: 04.11.1999 JP 31363399
(71) Applicant: Center for Advanced Science and Technology Incubation, Ltd., Tokyo 100-0005 (JP)
(72) Inventor: KARUBE, Isao, Kawasaki-shi, Kanagawa 216-0002 (JP); IKEBUKURO, Kazunori, Setagaya-ku, Tokyo 155-0033 (JP); YANO, Kazuyoshi, Setagaya-ku, Tokyo 155-0031 (JP); MIYACHI, Hirotaka, Musashimurayama-shi, Tokyo 208-0035 (JP)
(74) Representative: Warcoin, Jacques
(86) International application number: JP0007736
(87) International publication number: WO0133227

(57) **Abstract**

A method for immobilizing substances by formation of plasma-polymerized films, after the arrangement of the substances to be immobilized on the surface of a support, and the immobilized substances according to the method are provided. The immobilized substances are retained with use of plasma-polymerized films, thereby damage to the immobilized substances is minimized, and the liberation of the substances after the immobilization will hardly occur. They can be further applied for indirect immobilization of biotinylated polynucleotides, because binding activities of the substances are kept on the surface. In addition, proteins having binding activities such as antibodies can be immobilized to be used for the detection of ligands.

## Description

### Technical Field

The present invention relates to a method for immobilizing substances.

### Background Art

Artificial immobilization of substances on an appropriate support is widely performed, where the substances exist dissolved or dispersed in a liquid under natural conditions. For example, in an analysis utilizing the affinity between substances, bound substances and unbound substances are separated with use of a support. By way of example, in an analysis utilizing the binding between nucleic acid molecules comprising complementary nucleotide sequences, or hybridization, the nucleic acid molecules of interest are captured by the probe made of nucleic acids immobilized on a solid support. The captured nucleic acid molecules can be easily recovered by separation of the solid support from the liquid phase.

Similar separation steps are applied in an immunoassay utilizing antigen-antibody reactions. For example, in ELISA, which is typical kind of immunoassay, antibodies that recognize the antigens of interest contained in a sample are immobilized on a solid support, and the captured antigens on the solid phase are detected with enzyme-labeled antibodies. The sample after the reaction and the enzyme-labeled antibodies that were not used in the reaction can be easily removed by separation of the liquid phase from the solid support.

The binding between substances is utilized also in the purification of substances. For example, immobilization of one substance of the pair forming the binding reaction allows capture of the other substance of the pair and the washing away of other contaminating components to purify the substance. For example, when a liquid containing IgG is passed through a column filled with a resin on which protein A is immobilized, IgG will be adsorbed. Then, the resin is washed, and contaminants that are not bound to protein A are removed to leave only IgG in the column. Next, under specific conditions that will dissociate protein A - IgG binding , purified IgG may be recovered. This purification principle is widely utilized as affinity chromatography in purification of various substances.

The methods for immobilizing substances are useful also in the production of various substances based on chemical or biochemical reactions. Although enzymes and microorganisms are used in the production of a wide variety of substances, multiple purification steps are needed to recover the target substances from a reaction medium that contains a number of substrates and reaction products. Therefore, a technique comprising immobilizing a catalyst component such as an enzyme on a solid support and such, contacting it with a substrate, and inducing the desired enzymatic reaction has been developed. Immobilizing and recycling of enzymes are necessary from an economic point of view, because expensive enzymes might be lost during purification.

Immobilization of catalyst components such as an enzyme has become increasingly important with the commercialization of biosensors. The function of the biosensors is implemented by immobilizing various biocatalyst components or biochemical binding reaction components on a surface of the biosensors, and by detecting the interactions with the substance of interest by the biosensor. The components immobilized on the surface of the biosensors must stably retain activities needed for the reaction with the substance of interest. Also, the surface of the biosensors must be stable to avoid the transmission of unnecessary signals to the biosensor. In case the biosensor is used repeatedly, or it must be contacted continuously with the substance of interest, it is desirable that the liberation of the substance once immobilized would not occur. Thus, the immobilization of substances for biosensors needs to solve a number of problems.

As the methods for immobilizing substances that are widely applied in a various aspects, physical adsorption, immobilization by entrapment, and immobilization by chemical binding reaction are mainly known. As the physical adsorption, adsorption of proteins on the surface of hydrophobic resins is included. Antibody-immobilized plates utilized in ELISA are generally produced with this method. In physical adsorption, although operations for the immobilization are very easy, there are problems such as nonspecific adsorption and tendency to liberation of the adsorbed substances.

Immobilization by entrapment is a method in which immobilization is achieved by entrapping substances to be immobilized in a support such as a gel and a polymer. This method can be applied to a wide variety of substances, although the reactivity might be decreased because the active substances exist inside the support, or the separation of the reaction solution incorporated into the support might be inadequate. Also, the substances that are simply entrapped in the support can be liberated on a long-term basis, as is the case with physical adsorption.

In immobilization based on chemical binding, a method in which functional groups introduced on the surface of the support are chemically bound to functional groups of the substances to be immobilized is adopted. In this method, strong covalent bonds can be achieved, thereby a possibility of liberation can be minimized. However, a possibility of structural changes of the substances to be immobilized because of chemical reactions, and relative complexity of the reactions for immobilization are unavoidable problems. Further, it is difficult to apply to a wide variety of substances because functional groups that can be introduced on the surface of the support or functional groups usable for binding of the substances to be immobilized are limited by themselves.

In order to solve the various problems associate with these immobilization methods, many attempts have been reported. Utilization of plasma-polymerized films is one of those attempts. A wide variety of functional groups can be introduced on the surface of plasma-polymerized films by selecting a monomer gas as a starting material. Thus, the emergence of plasma-polymerized films has expanded the range of the possibilities of immobilization based on chemical binding.

For example, in surface plasmon resonance (hereinafter, abbreviated as SPR) biosensor (Unexamined Published Japanese Application No. (JP-A) Hei 9-264843) and quartz crystal oscillator biosensor (K. Nakanishi, et al. A novel method of immobilizing antibodies on a quartz crystal microbalance using plasma-polymerized films for immunosensors. Anal. Chem. 1996,68:1695-1700), functional groups that are introduced on the surface of the biosensor by plasma-polymerized films are utilized for immobilization of proteins. In these prior arts, although plasma-polymerized films are utilized, ultimate immobilization depends on a chemical binding, and problems such as damage of the substances to be immobilized cannot be solved.

In addition, a method of retaining the enzyme inside an ultrafilter membrane and coating its surface with plasma-polymerized films is known (Kikuko Yoshimura, et al., "Preparation of enzyme-immobilized membrane for biosensors by plasma polymerization", Bunsekikagaku, 1990, 39:749-753). In this method, a porous support is necessary, and the ultimate form of the immobilized substances is limited to a membrane. Additionally, both sides of the membrane must be coated. Further, since the substances to be immobilized are entrapped inside the membrane, reactions with immobilized substances cannot be expected on the outside surface of the plasma-polymerized films, and reactions mainly occur inside the support. There is another report utilizing plasma-polymerized films for immobilization by entrapment of lipase (JP-A Hei 6-153971), in which substances are also included inside the support. Therefore, the substances inside the support cannot be entrapped in coupling reactions and such on the surface of the immobilized substances.

Compounds that are desired to be immobilized on a support include polynucleotides. For example, DNA microarrays, which receives attention as a tool for analyzing genes, comprise a glass slide on which many kinds of polynucleotides are immobilized in high density. Presently, as a method of immobilization of polynucleotide, a method of attaching previously prepared polynucleotides to a glass slide by spotting the polynucleotides onto the glass slide, and a method of synthesizing nucleotides by elongation with one base after another on the slide have been commercialized. In the method of spotting polynucleotides, a phenomenon that polynucleotides are adsorbed to the surface of a glass slide that is coated with a basic protein such as poly-L-lysine is utilized for the immobilization of polynucleotides. This immobilization method can be easily mechanized, and thus, a number of DNA microarrays can be fabricated efficiently.

However, DNA microarrays obtained by adsorption of the polynucleotides on the slide are often associated with nonspecific adsorption of labeled polynucleotides leading to a disturbance of highly sensitive analysis. Also, adsorbed polynucleotides, which cannot avoid loss accompanying operations such as washing, would inhibit the improvement of the sensitivity. In the method of chemically synthesizing oligonucleotides on the support, the polynucleotides, which are immobilized by covalent bonds, are hardly liberated from the support. However, highly controlled and complicated chemical reactions are needed to synthesize many kinds of nucleotide sequences on the solid phase.

Thus, DNA arrays in which polynucleotides can be immobilized in a simple manner, nonspecific attachment are hard to occur, and polynucleotides once immobilized would not be liberated are desired.

In addition to polynucleotides, proteins including antibodies and protein libraries are also included in the substances that are desired to be immobilized on a support. Because the sequencing of the human genome has almost been completed, the analysis of proteins expressed according to the genome information, that is, the analysis of proteomes will become most important. In the analysis of proteomes, because the finding of how the individual proteins are expressed and how they interact with one another is important, the development of a chip with which many proteins can be detected simultaneously and efficiently is needed.

For this purpose, it may be effective to utilize a chip on which many kinds of proteins are immobilized. The most important step in the development of a chip is immobilization of proteins, and up to now, a variety of techniques such as adsorption and covalent binding have been tested, but the efficient immobilization of many kinds of proteins in high density was found to be very difficult.

### Disclosure of the Invention

An object of the present invention is to provide a method for immobilizing substances, in which the immobilized substances are hard to be liberated, and damage to the substances to be immobilized would be minimized. Another object of the present invention is to provide a method for immobilizing substances, which can be performed by simple operations and is applicable to the immobilization of a wide variety of substances.

Even if the functional groups can be selected without restraint, damage to the substances to be immobilized cannot be avoided as long as the immobilization depends on a chemical binding. Therefore, the present inventors considered the use of plasma-polymerized films. The plasma-polymerized films can be formed into highly uniform thin films in a desirable form under moderate temperature and pressure. Then, after the substances to be immobilized are arranged on the surface of the support, the plasma-polymerized films are formed, which are able to retain the substances on the surface of the support. Furthermore, the present inventors found that activities of the substances retained on the surface of the plasma-polymerized films are maintained and completed the present invention. The inventors showed that a monomer material supplied in the form of gas becomes an active species by plasma energy, although the substances to be immobilized arranged on the surface of the support are hardly affected. Namely, the present invention relates to a method for immobilizing substances described below, the immobilized substances obtained by this method, and their use:
(1) a method for immobilizing a substance on a surface of a support, said method comprising the following steps of:
   a) arranging a first substance to be immobilized on the surface of the support; and
   b) forming a plasma-polymerized film on the surface of the support after step a) ;
(2) the method according to (1), wherein the first substance is a protein;
(3) the method according to (2), wherein the protein has a binding activity;
(4) the method according to (3) , wherein the protein is selected from the group consisting of avidin, streptavidin, lectin, protein A, protein G, an antibody, a receptor, a DNA binding protein, and a derivative of them;
(5) the method according to (4), wherein the protein having a binding activity is an antibody or a fragment comprising an antigen binding domain thereof;
(6) the method according to (5), wherein the thickness of the plasma-polymerized film is 30 to 120 Å;
(7) the method according to (3) , further comprising, after step b), the step of:
   c) binding, to the protein having a binding activity, a second substance modified with a ligand of said protein;
(8) the method according to (7), wherein the second substance is selected from the group consisting of a protein, a sugar, a polynucleotide, a hormone, and a physiologically active substance;
(9) the method according to (8), wherein the second substance is a polynucleotide;
(10) the method according to (9), wherein the first substance is avidin and/or streptavidin, and the ligand that modifies the second substance is biotin;
(11) the method according to (9), wherein polynucleotides having different nucleotide sequences are bound to each of the defined areas on the support;
(12) the method according to (9), wherein the plasma-polymerized film has a hydrophobic surface;
(13) the method according to (1), wherein the plasma-polymerized film is formed in advance on the surface of the support in step a);
(14) the method according to (13), wherein the plasma-polymerized film formed in advance has a hydrophobic surface;
(15) the method according to (2), wherein the protein has an enzyme activity;
(16) an immobilized substance obtained by the method of (1);
(17) an immobilized substance obtained by the method of (5);
(18) an immobilized substance obtained by the method of (9);
(19) an immobilized substance comprising the following components:
   a) a first substance arranged on a surface of a support; and
   b) a plasma-polymerized film retaining the first substance;
(20) the immobilized substance of (19), wherein the first substance is a protein;
(21) the immobilized substance of (20), wherein the protein has a binding activity;
(22) the immobilized substance of (21), wherein the protein is selected from the group consisting of avidin, streptavidin, lectin, protein A, protein G, a DNA binding protein, and a derivative of them;
(23) the immobilized substance of (22), wherein the protein is an antibody;
(24) the immobilized substance of (23), wherein the thickness of the plasma-polymerized film is 30 to 120 Å;
(25) the immobilized substance of (21), wherein, to the protein having a binding activity, a second substance modified with a ligand of said protein is bound;
(26) the immobilized substance of (20), wherein the protein has an enzyme activity;
(27) an immobilized polynucleotide comprising the following components:
   a) a protein having binding activities, said protein arranged on a surface of a support;
   b) a plasma-polymerized film retaining the protein having a binding activity; and
   c) a polynucleotide immobilized on the surface of the support through a ligand of the protein having a binding activity;
(28) the immobilized polynucleotide of (27), wherein polynucleotides having different nucleotide sequences are bound to each of the defined areas on the support;
(29) a carrier for immobilizing a polynucleotide, wherein a protein having binding activities is retained on the surface of a support with a plasma-polymerized film;
(30) a kit for immobilizing a polynucleotide, said kit comprising the carrier of (29) and a primer modified with a ligand of the protein having a binding activity;
(31) a method for nucleic acid hybridization assay, said method comprising the following steps of:
   a) contacting a sample comprising a nucleic acid to be detected, with the immobilized substance of (18) or the immobilized polynucleotide of (27); and
   b) detecting hybridization of a probe to the nucleic acid in the sample; and
(32) a method for detecting a ligand, said method comprising the following steps of:
   a) contacting a sample comprising a ligand to be detected, with the immobilized substance of (17) or (23); and
   b) detecting the binding of an antibody or a fragment comprising an antigen binding domain thereof to the ligand in the sample.

Alternatively, the present invention relates to a use of a carrier in which proteins having binding activities are retained on the surface of the support with plasma-polymerized films, in immobilization method of polynucleotides. Further, the present invention relates to a use of a carrier in which proteins having binding activities are retained on the surface of the support with plasma-polymerized films, in detection of ligands.

In the present immobilization method, the first substance to be immobilized is arranged on the surface of the support. Arranging substances means literally that substances are placed in any form on the surface of a support. Therefore, especially strong adsorption or chemical binding between them is not necessarily needed. Specifically, the first substance to be immobilized is dissolved (or dispersed) in an appropriate solvent, and the substance may be arranged by applying it on the support, or by immersing the support in the solution and then drying it. Preferably, the solvent used in this procedure is dried sufficiently prior to the plasma polymerization. If the solvent remains on the surface of the support, it might interfere with the formation of uniform plasma-polymerized films. Additionally, solid substances may be heat-melted to be arranged by applying or spraying them onto the support.

The first substance to be immobilized according to the present invention may be any compound that would not be vaporized in the field of plasma polymerization reaction. Specifically, a wide variety of substances such as proteins, sugars, nucleic acids and such can be immobilized according to the present invention. The substances that are particularly advantageous as the first substance to be immobilized according to the present invention are those that are expected to express activities on the surface of the plasma-polymerized films. These activities include for example binding activities and enzyme activities.

When the substance having binding activities is immobilized as the first substance, ligands of the first substance can additionally be bound. Binding activities with the ligands can be utilized not only in detection and purification of the ligands, but also in immobilization of the substance modified with the ligands (the second substance). The use of binding with the ligands is described more specifically below.

On the other hand, when the substance having enzyme activities is immobilized as the first substance, it can be used as an immobilized enzyme, a catalyst for bioreactors, or an enzyme biosensor. In the present invention, the substance to be immobilized that has enzyme activities can be not only a single kind of enzyme but also a mixture of two or more kinds of enzymes.

In the present invention, a combination of the substances where binding is observed may be described as binding partners. In order to be bound together, it is important for binding partners to come closer. In the present invention, in which the activities of the substance to be immobilized are maintained on the surface of plasma-polymerized films, binding activities would be maintained on that surface. That is, conditions that are very advantageous for the substances to come closer can be achieved. For example, it is known that bindings are observed in the combinations of substances listed below:
avidin (or streptavidin) - biotin;
sugar - lectin;
histidine tag - metal ion;
protein A (or protein G) - IgG;
antibody - antigen;
hormone - hormone receptor; and
DNA binding protein - DNA.

When binding partners are used as the first substance to be immobilized, the compound to be immobilized is not particularly limited. Namely, any one of the binding partners can be immobilized as the first substance. However, since small molecules may be covered with plasma-polymerized films, leading to difficulty to maintain binding activities on that surface, it is advantageous to use relatively large molecules such as proteins as immobilized substances. However, the present invention does not exclude immobilization of small molecules. Even with small molecules, if plasma-polymerized thin films in which the molecules are not completely entrapped are formed, it is possible to maintain the activities of the immobilized substances on the surface of the films. According to the disclosure of the present invention, those skilled in the art can adjust the conditions of the plasma polymerization to allow maintaining the activities of the substances to be immobilized on the surface of the films.

For the reasons described above, in the present invention, for example, proteins having binding activities are preferred as the first substance to be immobilized. Avidin (or streptavidin) is a protein that forms a strong binding with biotin. Lectin is a generic name for sugar binding proteins, and is bound with a sugar or a sugar chain. Protein A and protein G are known as proteins that are bound with the constant region of IgG. As for hormone - hormone receptor, the structures in a large number of combinations have already been shown. Hormone receptors compose desirable proteins having binding activities in the present invention. As DNA binding proteins, proteins associated with gene repair system, which would recognize a mismatch of a double stranded DNA and would be bound to it, such as MutS and MutM, are known. In addition, a transcription factor involved in transcriptional regulation of genes is also one of the proteins that recognize a DNA nucleotide sequence and is bound to it. Derivatives of these proteins having binding activities can be utilized as the first substance to be immobilized. In the present invention, derivatives mean molecules that have been modified in their structure while maintaining the activities. Specifically, for example, fragments consisting only of binding domains that are necessary for the expression of binding activities, or fusion proteins of the above fragments and other proteins can be listed as the derivatives.

For the support on which the substance to be immobilized is arranged, any material can be used as long as it is possible to arrange the substance to be immobilized on its surface. Therefore, a material may be selected according to the ultimate usage of the immobilized substance. Usually, a material that has a specific strength to support ultrathin plasma-polymerized films, and that is nonpermeable to arrange the substance to be immobilized on its surface is used.

A material for solvent-nonpermeable support that is usable in the present invention specifically includes glass, metal, silicon, or plastic. However, a porous material through which the substance to be immobilized cannot permeate and through which solvent can permeate may be utilized in the present invention. Moreover, as long as the activities of the immobilized substance can be maintained on the surface of the plasma-polymerized films ultimately formed, a porous support like a filter can be utilized. In the present invention, the surface of the support may be coated with plasma-polymerized films in advance. According to our findings, the arrangement of the substance to be immobilized and the ultimate formation of plasma-polymerized films after the coating with plasma-polymerized films in advance can produce immobilized substance with higher activities. The plasma-polymerized films formed in advance may be materials that easily adsorb the substance to be immobilized. When the support is coated with plasma-polymerized films before the substance to be immobilized is arranged, the surface of the support can become solvent-nonpermeable regardless of its material.

The form of the support can be arbitrarily selected according to its usage. With plasma-polymerized films, even a surface having a complicated form can be coated with uniform thin films. Thus, the form of the support can be optional. For example, the substance can be immobilized on the inner wall of a container. Also, the substance can be immobilized on the surface of microparticles or spheres of macroscopic size. Such a three dimensional support is advantageous for use in the packing of a column and such. When used as a support for a DNA array, a standard glass slide may be used.

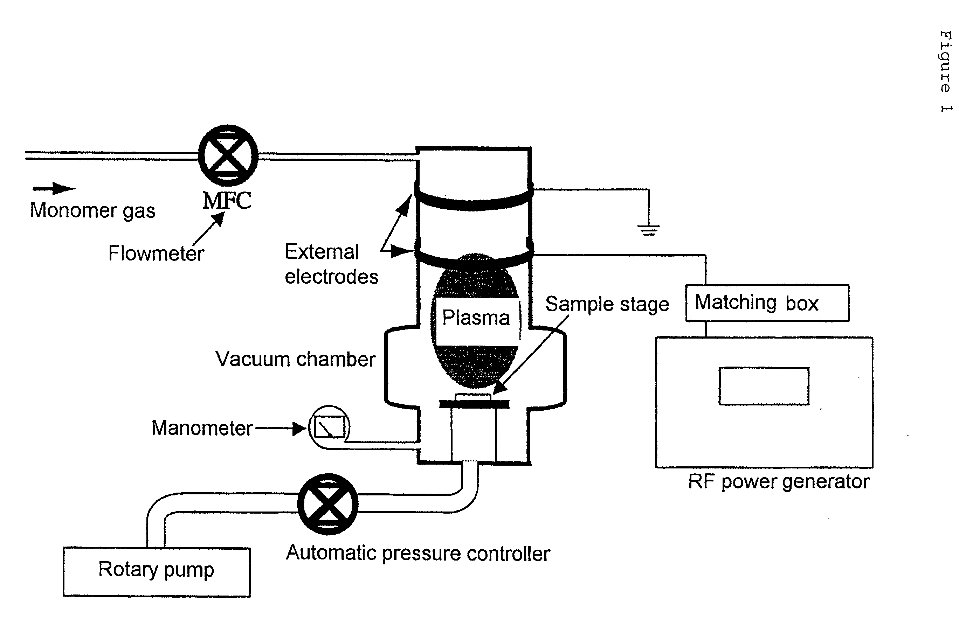

Plasma polymerization is a technique to form a membrane directly on the surface of a support by plasma excitation of a monomer gas *in vacuo*. Figure 1 shows the structure of the plasma polymerization apparatus used in the examples. Plasma-polymerized films having various characteristics can be obtained by changing the component of the monomer gas. In plasma polymerization, polymerization is possible using any monomer in principle. This is because, in order to obtain usual polymers, it is necessary to cleave double bonds, but in plasma, a monomer gas gives a large number of active species through which polymerization reactions occur. Since plasma energy is used for the polymerization, effect on the proteins in the reaction field can be minimized. Thus, the activities of the proteins will be maintained in a high level.

Monomer gases for plasma-polymerized films in the present invention is any one of those provide polymerized films that can retain the substance to be immobilized on the surface of the support. In the present invention, retaining the substance on the surface of a support means a state in which the substance to be immobilized is captured in the structure of plasma-polymerized films. Thus, the first immobilized substance will be sealed in the plasma-polymerized films on the surface of the support. Consequently, the liberation of the first immobilized substance will be effectively inhibited.

On the other hand, in the present invention, at least a part of the substance to be immobilized is in a state in which the active sites can express their activities on the outer surface of the plasma-polymerized films. Therefore, the immobilized substance of the present invention is distinct from the immobilized substance in which the substance to be immobilized is chemically bound to functional groups on the surface of the plasma-polymerized films. Also, it has a clearly different structure from that of the known immobilized substance in which the substance to be immobilized is permeated into a porous material and its surface is coated with the plasma-polymerized films.

Monomer gas giving plasma-polymerized films that satisfy these conditions includes the following ("Plasma polymerization", ed. Yoshihito Nagata, written by Mitsuo Kakuta, Kaoru Nakajima, Masataka Miyamura, Shinzo Morita, et al., Tokyo Kagaku Dozin, 1986).

Alkanes or cycloalkanes include the following compounds:
methane, ethane, propane, butane, isobutane, pentane, isopentane, neopentane, hexane, isohexane, 3-methylpentane, 2,2-dimethylbutane, 2,3-dimethylbutane, heptane, 2,2,3-trimethylbutane, octane, nonane, decane, methane-d1, methane-d2, methane-d3, methane-d4, cyclopropane, cyclobutane, cyclopentane, cyclohexane, methylcyclohexane, cyclooctane, cis-decalin, and trans-decalin.

Alkenes, alkynes, or cycloalkynes include the following compounds:
ethylene, propylene, 1-butene, (Z)-2-butene, (E)-2-butene, 2-methylpropene, 1-pentene, 2-methyl-1-butene, 3-methyl-1-butene, 2-methyl-2-butene, 1-hexene, (E)-2-hexene, (E)-3-hexene, 3-methyl-1-pentene, 2,3-dimethyl-2-butene, 1-heptene, 1-octene, (E)-2-octene, 1-decene, 1,3-butadiene, (Z)-1,3-pentadiene, (E)-1,3-pentadiene, isoprene, 2,3-dimethyl-1,3-butadiene, acetylene, propyne, 1-butyne, 2-butyne, 1-pentyne, 3-methyl-1-butyne, vinylacetylene, cyclopropene, cyclobutene, cyclopentene, cyclohexene, cycloheptene, cyclopentadiene, 1,3-cycloheptadiene, and cyclooctatetraene.

Alcohols, aldehydes, ketones, carboxylic acids, or esters include the following compounds:
methanol, ethanol, 1-propanol, 2-propanol, 1-butanol, 2-butanol, 2-methyl-1-propanol, 2-methyl-2-propanol, allyl alcohol, 1,3-butanediol, 2,3-butanediol, formaldehyde, acetaldehyde, propionaldehyde, butylaldehyde, valeraldehyde, isovaleraldehyde, acrylaldehyde, crotonaldehyde, glyoxal, acetone, 2-butanone, 2-pentanone, 3-methyl-2-butanone, 3-pentanone, 2-hexanone, 4-methyl-2-pentanone, 2-heptanone, cyclobutanone, cyclopentanone, cyclohexanone, cycloheptanone, cyclooctanone, 4-methyl-3-penten-2-one, 2,3-butandione, formic acid, acetic acid, propionic acid, butyric acid, isobutyric acid, acrylic acid, methyl formate, ethyl formate, propyl formate, butyl formate, isobutyl formate, methyl acetate, ethyl acetate, propyl acetate, isopropyl acetate, butyl acetate, isobutyl acetate, s-butyl acetate, methyl propionate, methyl butyrate, vinyl acetate, and allyl acetate.

Compounds usable as monomer gases such as ethers, amines, or the like include the following:
dimethyl ether, diethyl ether, dipropyl ether, diisopropyl ether, dibutyl ether, ethylene oxide, 1,3-dioxolane, 1,3-dioxane, 1,4-dioxane, methyl vinyl ether, methylamine, ethylamine, propylamine, isopropylamine, butylamine, isobutylamine, s-butylamine, t-butylamine, dimethylamine, trimethylamine, diethylamine, triethylamine, dipropylamine, diisopropylamine, tripropylamine, dibutylamine, allylamine, formamide, acetamide, N-methylacetamide, N,N-dimethylformamide, N,N-dimethylacetamide, methanethiol, ethanethiol, dimethyl sulfide, diethyl sulfide, dipropyl sulfide, dimethyl disulfide, diethyl disulfide, methanedithiol, 1,2-ethanedithiol, nitromethane, nitroethane, 1-nitropropane, 2-nitropropane, 1-nitrobutane, 2-nitrobutane, acetonitrile, propionitrile, and acrylonitrile.
Also, the following halides can be used as monomer gases:
   fluoromethane, difluoromethane, fluoroform, tetrafluoromethane (carbon tetrafluoride), vinyl fluoride, 1,1-difluoroethylene, (Z)-1,2-difluoroethylene, (E)-1,2-difluoroethylene, trifluoroethylene, tetrafluoroethylene, 1,1,4,4-tetrafluorobutadiene, perfluorobutadiene, 2-fluoroethanol, trifluoroacetic acid, 1,1,1-trifluoro-2-propanone, perfluoroacetone, chloromethane, dichloromethane, chloroform, tetrachloromethane (carbon tetrachloride), chloroethane, 1,1-dichloroethane, 1,2-dichloroethane, 1-chloropropane, 2-chloropropane, 1,2-dichloropropane, 1,3-dichloropropane, 1-chlorobutane, 2-chlorobutane, 1-chloro-2-methylpropane, 2-chloro-2-methylpropane, chlorocyclopropane, 1,1-dichlorocyclopropane, vinyl chloride, 1,1-dichloroethylene, (Z)-1,2-dichloroethylene, (E)-1,2-dichloroethylene, trichloroethylene, tetrachloroethylene, 3-chloropropene, 1,3-dichloropropene, chloroacetylene, dichloroacetylene, 1-chloropropyne, 2-chloroethanol, chloroacetaldehyde, chloroacetonitrile, dichloroacetonitrile, trichloroacetonitrile, bromomethane, dibromomethane, bromoform, tetrabromomethane (carbon tetrabromide), bromoethane, 1,1-dibromoethane, 1,2-dibromoethane, 1-bromopropane, 2-bromopropane, 1,3-dibromopropane, 1-bromobutane, 2-bromobutane, 1-bromo-2-methylpropane, 2-bromo-2-methylpropane, 1,4-dibromobutane, 1-bromobicyclo[2.2.1]heptane, 1-bromobicyclo[2.2.2]octane, vinyl bromide, 3-bromopropene, 1,3-dibromopropene, bromoacetylene, dibromoacetylene, 1-bromopropyne, 2-bromoethanol, iodomethane, diiodomethane, iodoform, tetraiodomethane (carbon tetraiodide), iodoethane, 1-iodopropane, 2-iodopropane, 1-iodobutane, 2-iodobutane, 1-iodo-2-methylpropane, 2-iodo-2-methylpropane, 1-iodopentane, 3-iodopropene, iodoacetylene, diiodoacetylene, 2-iodoethanol, 1-bromo-2-chloroethane, 1,1,1-trifluoro-2-iodoethane, 2-chloro-1,1-difluoroethylene, 1-chloro-1,2,2-trifluoroethylene, 1,1-dichloro-2,2-difluoroethylene, 1-bromo-2-chloroacetylene, 1-chloro-2-iodoacetylene, and 1-bromo-2-iodoacetylene.

Further, the following aromatic hydrocarbons can be used as monomer gases:
benzene, toluene, ethylbenzene, propylbenzene, cumene, butylbenzene, s-butylbenzene, t-butylbenzene, o-xylene, m-xylene, p-xylene, o-diethylbenzene, m-diethylbenzene, p-diethylbenzene, mesitylene, 1,2,4,5-tetramethylbenzene, styrene, phenylacetylene, (E)-1-propenylbenzene, (E)-1-phenylbutadiene, 2-phenylbutadiene, biphenyl, naphthalene, 1-methylnaphthalene, 2-methylnaphthalene, anthracene, phenanthrene, pyrene, naphthacene, chrysene, and pentacene.

In addition, the following benzene derivatives are useful for monomer gases of the present invention:
phenol, benzaldehyde, acetophenone, anisole, benzylmethylether, aniline, benzylamine, thiophenol, benzonitrile, fluorobenzene, chlorobenzene, bromobenzene, iodobenzene, o-dichlorobenzene, m-dichlorobenzene, p-dichlorobenzene, o-dibromobenzene, m-dibromobenzene, p-dibromobenzene, trifluorobenzene, hexafluorobenzene, o-fluorotoluene, m-fluorotoluene, p-fluorotoluene, o-chlorotoluene, p-chlorotoluene, o-bromotoluene, p-bromotoluene, o-iodotoluene, m-iodotoluene, p-iodotoluene, p-chlorofluorobenzene, and o-chloroiodobenzene.

Also, the following heterocyclic compounds can be used as monomer gases:
pyridine, 2-methylpyridine, 3-methylpyridine, 4-methylpyridine, 2,6-dimethylpyridine, 2,5-dimethylpyridine, 2,4-dimethylpyridine, pyridazine, pyrimidine, pyrazine, 1,3,5-triazine, pyridine N-oxide, 2-methylpyridine N-oxide, 3-methylpyridine N-oxide, 4-methylpyridine N-oxide, 2,6-dimethylpyridine N-oxide, furan, methylfuran, tetrahydrofuran, pyrrole, pyrrolidine, thiophene, and 2-chlorothiophene.

In addition, troponoid compounds such as tropone and tropolone, and organometallic compounds represented by tetramethylsilane, tetramethyltin, and tetramethyllead can be used as monomer gases.

Conditions under which the plasma-polymerized films are formed with these monomer gases are known. Specifically, conditions such as, for example, flow velocity, electric discharge power, electric discharge time, and pressure are considered to be important as primary factors that would affect the repeatability of the plasma polymerization reactions. In plasma polymerization, optimal polymerization conditions must be established according to the apparatus and monomers. There is a report describing that if W/FM values (where W is electric discharge power, F is flow velocity, and M is molecular weight of the monomer) are the same, the qualities of the films are similar (Yasuda, Plasma Polymerization, Academic Press, New York, 1985).

Considering the monomer gas used and the thickness of plasma-polymerized films ultimately needed, those skilled in the art routinely adjust these conditions appropriately. Also, some literatures show the effects of various parameters on the characteristics of plasma-polymerized films (Surface and Coatings Technology 82:1-15,1996, Polymer Engineering and Science 37/7:1188-1194, 1997). In order to fabricate plasma-polymerized films with hexamethyldisiloxane, which is described below as an advantageous monomer gas when an object is the immobilization of polynucleotides, selection of the optimal conditions, for example in a range below, may give approximately 0 - 240 Å of plasma-polymerized films:
flow velocity: 0 - 50 cm³/min;
electric discharge power: 0 - 300 W;
pressure: 10⁻⁶ - 10 Torr; and
electric discharge time: 0 - 5 min.
(temperature: 0 - 100°C).

Alternatively, more desirable conditions to fabricate approximately 0 - 240 Å of plasma-polymerized films include the following conditions:
flow velocity: 10 - 50 cm³/min;
electric discharge power: 20 - 100 W;
pressure: 0.05 - 0.6 Torr; and
electric discharge time: 30 sec - 5 min.
(temperature: room temperature).

When the immobilized substance obtained by the present invention is a substance having binding activities, a ligand that is the binding partner can be bound to this immobilized substance. The binding of the ligand is made possible by the characteristic of the present invention in which the immobilized substance retains its activities on the surface of the plasma-polymerized films. Then, giving the second substance modified with the binding partner makes it possible to immobilize any second substance. Since the second substance immobilized through the ligand exists in a state in which the whole molecules are completely exposed on the'surface of the plasma-polymerized films, it will be advantageous for the contact with substances existing in a liquid phase. As a result, high reactivity can be expected in various reactions in a liquid phase in the present invention, compared to known methods such as, for example, immobilization by entrapment in which the substance to be immobilized is buried in the support.

Immobilization of the second substance with use of a binding partner according to the present invention is specifically described below. As a method for immobilizing substances according to the present invention, utilizing binding partners, i.e. streptavidin - biotin as one of the useful combinations, immobilization method of polynucleotides (the second substance) may be shown. Namely, streptavidin (the first substance) is arranged on a support, for example, a glass slide, and then it is immobilized by formation of plasma-polymerized films. Streptavidin is arranged on the support by being applied as aqueous solution, followed by being dried *in vacuo* or air-dried. At this time, a glass slide that is undercoated with plasma-polymerized films in advance may be used. More reliable immobilization of streptavidin can be expected by undercoating with plasma-polymerized films.

On the surface of the glass slide on which streptavidin is arranged, plasma-polymerized films are formed. Then, the thickness of the plasma-polymerized films is made to be 0 - 240 Å, preferably 5 - 150 Å, more preferably 30 - 45 Å, and a binding activity of streptavidin to biotin can be maintained on the surface of the films. As a result, biotin is captured by a binding activity of streptavidin on the surface of the films. The thickness of the plasma-polymerized films is controlled by polymerization time. In order to obtain a suitable thickness of the films, those skilled in the art can adjust the polymerization conditions depending on the combination of the first substance to be immobilized and the plasma-polymerized films, according to the present invention.

The second substance modified with biotin can be bound to a glass slide on which streptavidin is immobilized. As the second substance, polynucleotides, for example, can be used. Polynucleotides are molecules formed by polymerization of a large number of nucleotides. In the present invention, the number of nucleotides that compose a polynucleotide is not specifically limited. When a polynucleotide is composed of not more than several tens of nucleotides, it is also called an oligonucleotide. On the other hand, when a polynucleotide is composed of more than several hundreds of deoxyribonucleotides, it is called a DNA. Polynucleotides or oligonucleotides of the present invention may be natural substances or chemically synthesized ones. Polynucleotides of the present invention include DNAs or RNAs. Polynucleotides of the present invention may be the ones synthesized by enzymatic reactions such as PCR based on template DNAs. A method for modifying polynucleotides with biotin is well known. For example, by incorporating biotinylated nucleotides in the synthesis of polynucleotides, polynucleotides having biotin bound at any position can be synthesized. Moreover, PCR using biotin-bound oligonucleotides as primers may give biotin-modified DNAs as amplified products in large quantities. Biotin-modified DNAs can be easily immobilized on the surface of a streptavidin-immobilized glass slide by contacting it with the slide according to the present invention.

The immobilized polynucleotides of the present invention thus obtained are useful as immobilized probes for hybridization assays. In particular, they are advantageous as a solid phase for hybridization, when a highly hydrophobic material is used as the plasma-polymerized films. Specifically, first, since they are hydrophobic, nonspecific adsorption of polynucleotides would hardly occur. Generally, in hybridization assays, analysis is performed based on signals of labeled probes captured to the solid phase by hybridization. Since nonspecific adsorption of polynucleotides may generate signals that are not related to hybridization, it may cause a decrease of the sensitivity through an increase of the background signal. On the surface of the hydrophobic plasma-polymerized films, nonspecific adsorption of polynucleotides unrelated to hybridization will hardly occur, and the background signals will be kept low, resulting in a high sensitivity.

Second, immobilized polynucleotides themselves become easy to be liberated from the surface of the plasma-polymerized films, and there may be an advantage that opportunities of hybridization with polynucleotides comprising complementary nucleotide sequences in a liquid phase would increase. On the other hand, in a known support for immobilization of polynucleotides, many of the immobilized polynucleotides are thought to be adhered to the surface of the support, and in such a condition, opportunities of hybridization with polynucleotides in a liquid phase might be limited. Such a characteristic is a great advantage of the immobilization method of the present invention when applied for immobilization of polynucleotides.

Among monomer gases described above, organosilicons containing silicon such as hexamethyldisiloxane (abbreviated as HMDS hereinbelow), tetramethyldisiloxane, and such, and hydrocarbon monomers such as alkane, alkyne, alkene, benzene, and xylene can be used to provide hydrophobic plasma-polymerized films. The hydrophilicity of plasma-polymerized films depends not only on the monomer gas used as the source material, but also on the conditions for polymerization. However, HMDS is one of the monomer gases that can provide hydrophobic films under a wide range of conditions.

Besides, in standard plasma polymerization, monomer gas is delivered with bubbling of argon, but it is better to form hydrophobic films without bubbling when HMDS is utilized in the present invention.

The immobilized polynucleotides according to the present invention are free from concerns of being liberated from a glass slide through operations such as hybridization and washing of the slide, because they are immobilized by very strong binding between streptavidin and biotin. Particularly in washing steps, because aqueous solution will not remain on the surface of hydrophobic plasma-polymerized films, there is no need to wash carefully to remove the reaction solution completely. This can be considered to be a very advantageous characteristic of the immobilized polynucleotides of the present invention in the sense that the loss of immobilized polynucleotides associated with washing steps is minimized.

In one embodiment of the immobilized polynucleotides of the present invention, many kinds of polynucleotides having different nucleotide sequences can be immobilized on the surface of a glass slide. These immobilized polynucleotides are referred to as DNA arrays. Among these, those in which a large quantity of polynucleotides, of several thousands to up to several tens of thousands in kind, is arranged in high density are called DNA microarrays. In order to fabricate DNA microarrays according to the present invention, PCR using primers modified with biotin may be performed for the synthesis of the polynucleotides to be immobilized. For example, amplification of inserts is performed with primers comprising nucleotide sequences of the vector, using, as templates, clones isolated from a cDNA library. Modification of primers with biotin enables all the amplification products to have biotin, and they can be bound to streptavidin immobilized prior to amplification.

In spotting many kinds of polynucleotides on a limited area, an automated apparatus called a DNA arrayer can be used. A DNA arrayer is an apparatus for sampling given quantity of the solution containing DNA to be spotted and for spotting it on a defined area on a glass slide automatically and rapidly. For example, a DNA arrayer that can arrange dots comprising spots having a diameter of 100 µm with intervening spaces of 10 µm on a glass slide or a membrane filter is commercially available (GMS417, Takara Shuzo).

A streptavidin-immobilized glass slide that is needed to fabricate DNA microarrays according to the present invention can be supplied as a support for the immobilization of polynucleotides. Alternatively, it can be further combined with biotinylated primers for amplification of immobilized probes to produce a kit for fabrication of DNA microarrays. To a kit for fabrication of DNA microarrays according to the present invention, a cDNA clone isolated from, for example, a cDNA library, or a vector for isolation of cDNA clone can be added. Using such a kit, users can easily construct a cDNA library from a source material prepared by themselves, and fabricate DNA arrays with probes prepared by a cDNA contained in this cDNA library. DNA arrays thus fabricated are useful for making gene expression profiles in a variety of cells.

DNA microarrays according to the present invention can be used in hybridization assays similarly to conventional DNA microarrays. Namely, they are contacted with fluorescence labeled sample, and after removing unreacted components, the fluorescent spots are observed with a fluorescence microscope and a scanner. A glass slide may be a suitable material for a support to be used in such a way, because a glass slide does not have fluorescence. In the present invention, since plasma polymerization films will coat a glass, it is advantageous to form plasma polymerization films using material that has no fluorescence so as not to impair the fluorescent property of the glass. Because HMDS exemplified above does not emit fluorescence, it is an advantageous material for immobilization of polynucleotides in terms of fluorescence property.

The present invention can be used for immobilization of enzymes on an enzyme biosensor, in addition to immobilization of polynucleotides on a glass slide. Namely, first, as a support, the surface of an oxidation-reduction electrode is used in place of a glass slide. As the second compound to be immobilized, enzymes modified with biotin are used as a substitute for polynucleotides modified with biotin. Further, using antibodies instead of enzymes allows construction of immunosensors. Enzymes and antibodies can be biotinylated with commercially available biotinylating agents.

In the present invention, it is also possible to immobilize antibodies themselves as ligands. That is, as the first substance, IgG binding proteins such as protein A and protein G are used. Then, their ligand, IgG can be bound to achieve immobilization of IgG. IgG thus immobilized can be made in a state that is suitable for reactions with antigens on which variable region is oriented to a liquid phase.

In the present invention, a support on which IgG binding protein such as protein A is immobilized is useful for purification of IgG, or immuno affinity chromatography of antigens that are recognized by IgG. Additionally, it can be utilized as immobilized antibodies for an immunoassay of antigens that are recognized by IgG, and as immunosensors.

Furthermore, an antibody or its fragment containing an antigen binding domain can be immobilized as the first substance of the present invention. In this embodiment, an antibody that is immobilized as the first substance, or its antigen binding domain is directly bound with the ligand. When an antibody represented by IgG is used as the first substance of the present invention, the binding activity between the antibody and an antigen can be maintained at a high level, by making the thickness of plasma-polymerized films to be 20 - 120 Å, preferably 30 - 90 Å, and more preferably 30 - 60 Å. When a fragment containing an antigen binding domain is used as an antibody, the maintenance of the activity of the antibody can be expected by making the thickness of films thin according to the molecular weight of the fragment to be immobilized. The antibody molecules to be immobilized in the present invention are not limited to IgG, and may be any class of antibody molecules that can be bound to the ligands. Also, the antibody molecules to be immobilized in the present invention can be artificial antibody molecules obtainable from the fragments, or chimeric antibodies and CDR grafts, as long as they maintain the binding activity with the ligands.

The immobilized substance on which an antibody is bound as the first substance in the present invention can be used for detection of a ligand that is recognized by the antibody. Namely, the sample containing a ligand to be detected is contacted with the immobilized substance, and the ligand that is bound to the antibody is detected, thereby enabling detection of the presence of the ligand.

Alternatively, according to the present invention, antibodies can be immobilized. Antibodies include not only proteins, but also any compounds that can cause immune responses such as sugar chains, lipids, or various low molecular weight organic compounds. Antibodies immobilized according to the present invention are useful as immunosensors to perform purification and detection of the antibodies.

When antibodies are immobilized according to the present invention, nonspecific adsorption of proteins can be prevented by composing plasma-polymerized films with hydrophilic material. It is because not only antibodies but also proteins are generally hard to be adsorbed to a hydrophilic surface. As monomer gases that provide hydrophilic plasma-polymerized films, nitrogen containing organic compounds such as acetonitrile, pyridine, propylamine, methylamine, or triethylamine are used.

In the present invention, as substances to be immobilized, proteins having catalytic activities like enzymes, in addition to proteins having binding activities, can be immobilized. The proteins immobilized according to the present invention maintain catalytic activities on the surface of the plasma-polymerized films. Therefore, they are easy to contact with their substrates, and exhibit high levels of enzymatic activities. The enzymes immobilized according to the present invention are useful as reactive reagents or as catalysts for various enzyme biosensors. Because enzymes can be immobilized on a support of any form according to the present invention, they can be used for microchip biosensors that need fine construction.

On the other hand, with known methods in which the surface of ultrafilter membranes retaining enzymes is coated with plasma-polymerized films, only substrates that penetrate the membranes can be involved in the enzyme reaction, because the enzymes are included inside the membranes. Thus, the efficiency of the enzyme reaction is limited by the physical properties of the membrane. In addition, enzyme activities cannot be expressed on large molecules that cannot penetrate the membranes.

Any prior art references cited herein are incorporated herein by reference.

### Brief Description of the Invention

Figure 1 shows the configuration of the apparatus for plasma polymerization used in the Examples.

Figure 2 shows photographs of the nonspecific adsorption of polynucleotides on the immobilized polynucleotides of the present invention compared to the polynucleotides immobilized by the known method.

Figure 3 shows the structure of the support on which antibodies are immobilized according to the present invention, produced in Example 2.

Figure 4 shows the arrangement of the spots of the antibodies on the supports on which antibodies are immobilized according to the present invention, produced in Example 2.

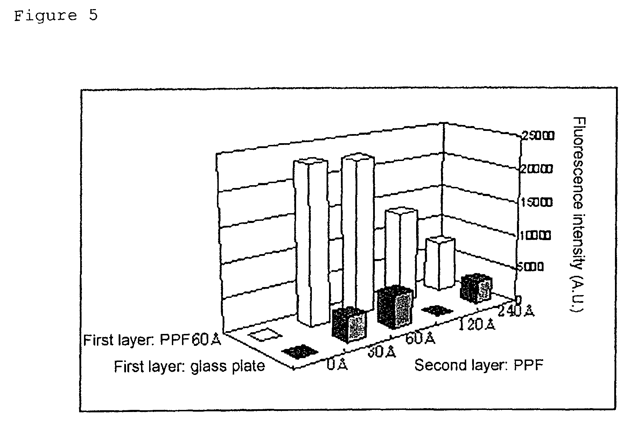

Figure 5 is a graph showing the effects of the presence of the plasma-polymerized films of the first layer and the thickness of the plasma-polymerized films of the second layer on the method for immobilizing antibodies according to the present invention. The fluorescence intensity (Au) is shown on the vertical axis, and the thickness of the plasma-polymerized films on the second layer is shown on the horizontal axis. The columns at the back indicated as "First layer: PPF 60 Å" show the results obtained when plasma-polymerized films with a thickness of 60 Å is formed on the first layer, and the columns in front indicated as "Second layer: glass plate" show the results obtained when antibodies are immobilized directly without plasma-polymerized films on the first layer.

Figure 6 shows the arrangement of the spots of the antibodies on the supports on which antibodies are immobilized according to the present invention, produced in Example 3. Anti-human serum albumin antibodies are spotted on the column indicated as Anti-HSA, and anti IgM antibodies are spotted on the column indicated as Anti-IgM.

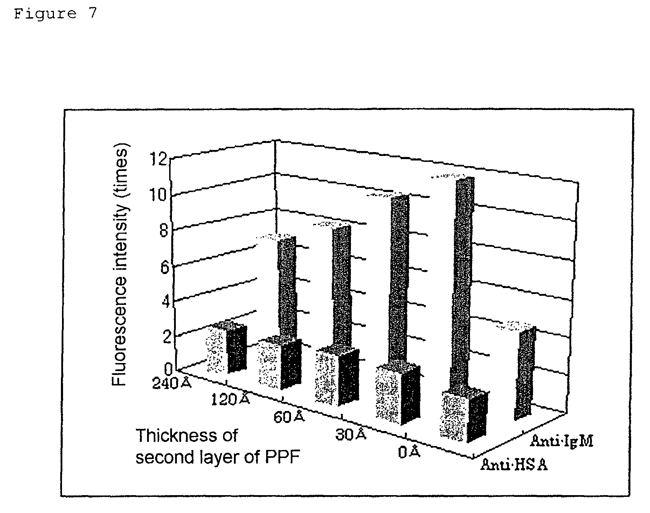

Figure 7 shows the reactivity of fluorescence labeled IgM with antibodies immobilized according to the present invention. The ratio of fluorescence intensity after the reaction to the fluorescence intensity before the reaction of the device with fluorescence labeled IgM is shown on the vertical axis, and the thickness of the plasma-polymerized films on the second layer is shown on the horizontal axis. The columns at the back indicated as Anti-IgM show the reactivity with anti-IgM antibodies, and the columns in front indicated as Anti-HSA show the reactivity with anti-human serum albumin antibodies.

### Best Mode for Carrying out the Invention

The present invention is described based on the examples more specifically below.

### [Example 1]

Based on the present invention, streptavidin was immobilized on glass supports, and oligonucleotides modified with biotin were immobilized further.

### a) Washing of the glasses

Glasses with a thickness of 0.7 mm produced by Dow Corning (7059) were used as supports. The glasses were immersed in heated hydrogen peroxide/aqueous ammonia/H₂O (1:1:8) for 30 minutes, and then washed repeatedly with excess amount of H₂O.

### b) Immobilization of streptavidin

The washed glasses were arranged on the sample stage of the apparatus for plasma immobilization, and the formation of thin films was carried out for 1 minute with radiowave power of 150 W, and a degree of vacuum inside the reactor of 0.3 Torr. Then, spots of 1 µl each of the 0.5 mg/ml solution of streptavidin (the solvent: H₂O) were spotted at specific positions, and dried at 4°C. These were arranged again on the sample stage of the apparatus for plasma immobilization, and the thin films were formed with similar conditions to those described above. However, the time of the plasma polymerization was 30 seconds at this time.

### c) Immobilization of oligo DNA

A solution of biotinylated oligo DNA was dropped at the position where streptavidin was arranged, and the oligo DNA was immobilized through the binding of streptavidin -'biotin (the biotinylated oligo DNA used in this example is described as the probe DNA hereinbelow). A 10 µM solution of the probe DNA dissolved in a buffer solution consisting of 10 mM Tris-HCl (pH 7.0), 10 mM EDTA, and 300 mM NaCl was dropped onto the streptavidin, and left at room temperature for 1 hour. Then the glasses were immersed in a phosphate buffer.

### d) DNA hybridization

DNA labeled with fluorescein at the 5' terminal (200 µM) was dissolved in a buffer solution consisting of 10 mM Tris-HCl (pH 7.0), 10 mM EDTA, and 300 mM NaCl, and DNA hybridization was carried out at 45°C for 1 hour.

### e) Washing

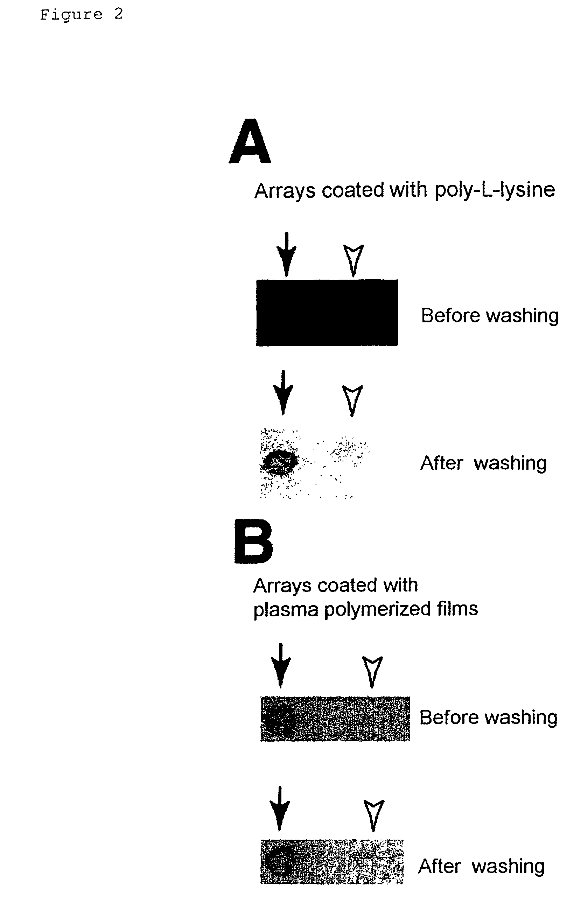

The arrays fabricated with the plasma polymerization method were washed by shaking in a solution of 0.2x SSC at room temperature for 10 minutes. The arrays coated with poly-L-lysine were washed in a solution of 1x SSC and 0.3% SDS (sodium dodecyl sulfate) for 5 minutes following hybridization, and then were washed in a solution of 0.2x SSC for 10 minutes. The washing was performed with a shaker at room temperature. Results are shown in Figure 2.

When DNAs were immobilized with the known method, spots could not be observed before the washing steps, and adequate washings were needed to identify the spots. Namely, it was found that inadequate washing might lead to nonspecific adsorption. On the other hand, when polynucleotides were immobilized according to the present invention, the reaction solution was removed nearly completely only by slanting the glass slide after the hybridization, and the fluorescence spots could be clearly identified without washing. Namely, it is obvious that the immobilized polynucleotides of the present invention will hardly cause nonspecific adsorption of polynucleotides.

### [Example 2]

In this example, antibodies that are most valuable as elements for identification of proteins were focused, and the object was to develop a chip on which antibodies are immobilized. First, a novel method for immobilizing antibodies with use of plasma polymerization was examined.

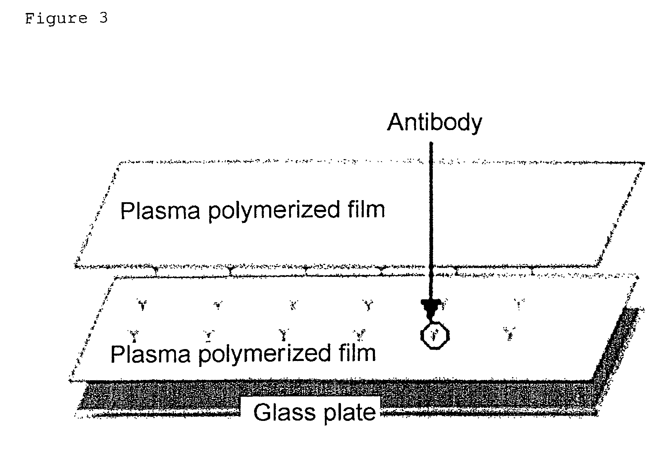

Prior to the immobilization of antibodies, polymer thin films were formed first with plasma polymerization on the surface of a glass plate, using hexamethyl disiloxane (HMDS) as a monomer. Next, antibodies were arranged on the plate by spotting, on which HMDS plasma-polymerized films were further formed to immobilize the antibodies between the films (Figure 3).

An apparatus shown in Figure 1 was used for plasma polymerization. The characteristics of the apparatus used in this example include a long distance between the electrodes and the stage. This might reduce the effect of electromagnetic waves on the biological samples.

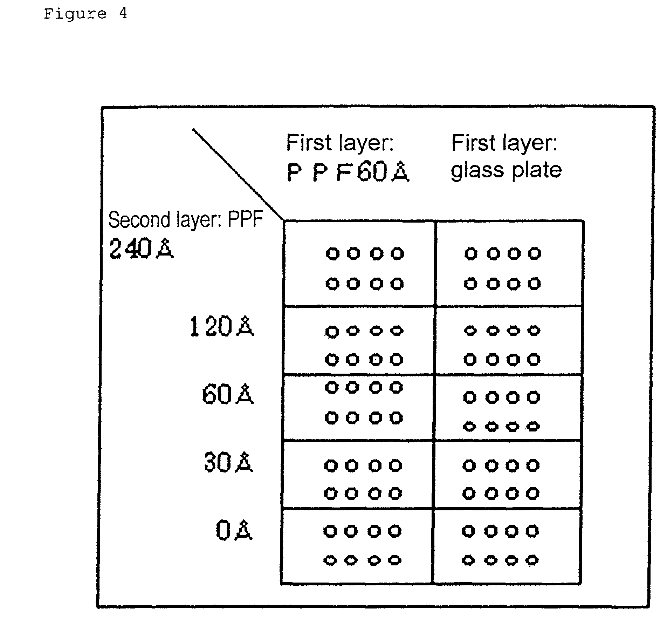

Then the optimal conditions for the immobilization of antibodies with plasma polymerization were examined by determining whether antigen - antibody reactions would actually occur. The glass plate was divided into 10 blocks as shown in Figure 4, and on each of which the antibodies were immobilized with different conditions.

First, the glass plate was divided into two areas on the right and the left, and plasma-polymerized films (PPF) with a thickness of 60 Å were formed only on the left side. The conditions for the formation of the films were as follows: a flow velocity of the monomer and pressure inside the reactor were adjusted to 18 ccm and 25 Pa (Detector: Pirani gauge), respectively; the pressure inside the reactor was kept at 40 Pa during the plasma polymerization reaction; and the thickness of the films was controlled according to the reaction time. Next, 1 µl of an 80 µg/ml solution of anti-human serum albumin IgG (ICN pharmaceuticals, Inc.) was spotted on each points with a pipetter.

After drying at 4°C over night, the second layer of plasma-polymerized films was formed. The conditions were adjusted in such a way that the thickness of the plasma-polymerized films was 0, 30, 60, 120, and 240 Å, as shown in Figure 4.

Then, the prepared device was immersed in 1 ml of a solution containing 120 µg/ml of fluorescence labeled human serum albumin (ICN pharmaceuticals, Inc.) at room temperature for 2 hours, washed with distilled water for 15 minutes, and then the fluorescence intensity was measured. A graph of the averaged values on each point corrected for the background is shown in Figure 5.

The columns at the back show the results obtained when the first layer of plasma-polymerized films was formed prior to the spotting of antibodies, and the columns in front show the results obtained when antibodies were spotted on the glass plate, each of which indicating the changes in the fluorescence intensity by the thickness of the second layer of plasma-polymerized films.

This graph shows that the fluorescence intensity became strongest when the first layer of plasma-polymerized films was present, and the thickness of the second layer of plasma-polymerized films was 30 - 60 Å. The higher fluorescence intensity in the presence of the first layer of plasma-polymerized films might be caused by the stronger binding with the hydrophobic sites of the antibodies, because plasma-polymerized films produced by HMDS as a monomer would be hydrophobic.

In addition, that the fluorescence intensity became higher when the thickness of the second layer of plasma-polymerized films was 30 - 60 Å is because the binding sites were left available, since the size of antibodies was estimated to be about 90 Å. Also, because the crystals may grow from the lower part when the plasma-polymerized films are formed, antibodies can be immobilized leaving the binding site available.

### [Example 3]

Next, that antigen-antibody reactions can be detected with antibodies immobilized according to the present invention was confirmed. As reagents, anti-human serum albumin IgG (ICN pharmaceuticals, Inc.), anti-human IgM IgG (CORTEX BIOCHEM, INC.), and fluorescence labeled IgM (CORTEX BIOCHEM, INC.) were used.

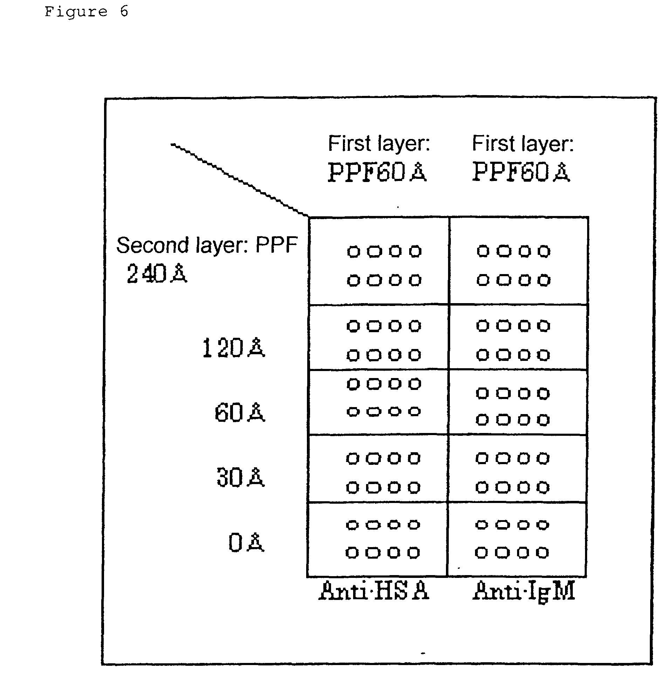

The device used was designed as shown in Figure 6. Namely, following the preparation of plasma-polymerized films with a thickness of 60 Å as the first layer, and 1 µl of an 80 µg/ml solution of anti-human serum albumin IgG (Anti-HSA) was spotted on the left side of the plate, and 1 µl of an 80 µg/ml solution of anti-human IgM IgG was spotted on the right side of the plate. After drying at 4°C over night, the second layer of plasma-polymerized films was formed. Similar to example 2, the polymerization conditions were adjusted in such a way that the thickness of the plasma-polymerized films was 0, 30, 60, 120, and 240 Å.

Then, the prepared device was immersed in 1 ml of a solution containing 100 µg/ml of fluorescence labeled IgM over night, washed with distilled water for 15 minutes, and then the fluorescence intensity was measured.

Results obtained from the measurement are shown in Figure 7.

In this graph, the ratio of fluorescence intensity after the reaction to the fluorescence intensity before the reaction of the device with fluorescence labeled IgM is shown on the vertical axis. The columns at the back shows the changes in the fluorescence intensities at the points at which anti-human IgM IgG (Anti-IgM) was spotted, where the change is due to the thickness of the second layer of plasma-polymerized films. The columns in front shows the changes in the fluorescence intensities at the points at which anti-human serum albumin IgG (Anti-HSA) was spotted, where the change is due to the thickness of the second layer of plasma-polymerized films.

As shown by the columns in front, the changes in the fluorescence intensity were not observed on the spots of Anti-HSA, but the fluorescent intensity changed according to the thickness of the films, when Anti-IgM was spotted. These results showed that the changes in immunological reactivity of antibodies according to the thickness of the films, indicated in the previous experiment, were shown as the changes in the fluorescence intensity. This graph shows that the immobilized antibodies react specifically with the antigens. In addition, in a reaction of IgM with Anti-IgM, it was found that the fluorescence intensity was highest when the thickness of the second layer of plasma-polymerized films was 30 - 60 Å. This result was conceivably obtained because the antibodies were immobilized on the plate, with the binding sites left available, since the size of antibodies was estimated to be about 90 Å.

This example showed that the immobilization of antibodies on a glass plate with the available binding sites left with plasma polymerization was possible. Also, because the obtained fluorescence intensity changed when the thickness of the second layer of plasma-polymerized films was changed, it was suggested that there should be some relationship between the size of antibodies and the thickness of the second layer of plasma-polymerized films. Further, it was found that the first layer of plasma-polymerized films acted advantageously because of the hydrophobic - hydrophobic interaction between the antibodies and the hydrophobic surface. The support on which antibodies are immobilized fabricated according to the present invention is useful for the specific detection of the antigens, i.e. ligands.

### Industrial Applicability

According to the present invention, immobilized substances in which the damage of the substances to be immobilized is minimized, and the substances will not liberated after immobilization can be obtained. The immobilized substances obtained according to the present invention retain binding activities and such of the immobilized substances on the surface of plasma-polymerized films. Therefore, when substances having binding activities such as, for example, streptavidin are immobilized, other substances can be indirectly immobilized with use of their ligand, biotin. This principle can be applied to polynucleotides to immobilize biotinylated polynucleotides on any sites on a solid phase easily and in high density. This characteristic is useful for the production of DNA microarrays.

In addition, the immobilized substances coated with plasma-polymerized films can be given any surface property by selection of a monomer gas that composes plasma-polymerized films. For example, by the formation of hydrophobic plasma-polymerized films, the surface property on which polynucleotides are hardly adsorbed can be given. Such immobilized substances allow the detection of signals based on the specific binding of polynucleotides, and implements a detection system with low background and high sensitivity.

Alternatively, the present invention is useful as a technique for immobilizing proteins on the surface of a support. According to the present invention, proteins can be easily, and certainly immobilized on the surface of a support. Furthermore, according to the present invention, damage to the proteins associated with the immobilization reactions is minimized, and high activities can be maintained even after the polymerization. These characteristics are useful for the immobilization of antibodies and a protein library. A support on which these proteins are immobilized is an important tool for an analysis of proteomes.

## Claims

1. A method for immobilizing a substance on a surface of a support, said method comprising the following steps of:
a) arranging a first substance to be immobilized on the surface of the support; and
b) forming a plasma-polymerized film on the surface of the support after step a).

2. The method according to claim 1, wherein the first substance is a protein.

3. The method according to claim 2, wherein the protein has a binding activity.

4. The method according to claim 3, wherein the protein is selected from the group consisting of avidin, streptavidin, lectin, protein A, protein G, an antibody, a receptor, a DNA binding protein, and a derivative of them.

5. The method according to claim 4, wherein the protein having a binding activity is an antibody or a fragment comprising an antigen binding domain thereof.

6. The method according to claim 5, wherein the thickness of the plasma-polymerized film is 30 to 120 Å.

7. The method according to claim 3, further comprising, after step b), the step of:
c) binding, to the protein having a binding activity, a second substance modified with a ligand of said protein.

8. The method according to claim 7, wherein the second substance is selected from the group consisting of a protein, a sugar, a polynucleotide, a hormone, and a physiologically active substance.

9. The method according to claim 8, wherein the second substance is a polynucleotide.

10. The method according to claim 9, wherein the first substance is avidin and/or streptavidin, and the ligand that modifies the second substance is biotin.

11. The method according to claim 9, wherein polynucleotides having different nucleotide sequences are bound to each of the defined areas on the support.

12. The method according to claim 9, wherein the plasma-polymerized film has a hydrophobic surface.

13. The method according to claim 1, wherein the plasma-polymerized film is formed in advance on the surface of the support in step a).

14. The method according to claim 13, wherein the plasma-polymerized film formed in advance has a hydrophobic surface.

15. The method according to claim 2, wherein the protein has an enzyme activity.

16. An immobilized substance obtained by the method of claim 1.

17. An immobilized substance obtained by the method of claim 5.

18. An immobilized substance obtained by the method of claim 9.

19. An immobilized substance comprising the following components:
a) a first substance arranged on a surface of a support; and
b) a plasma-polymerized film retaining the first substance.

20. The immobilized substance of claim 19, wherein the first substance is a protein.

21. The immobilized substance of claim 20, wherein the protein has a binding activity.

22. The immobilized substance of claim 21, wherein the protein is selected from the group consisting of avidin, streptavidin, lectin, protein A, protein G, a DNA binding protein, and a derivative of them.

23. The immobilized substance of claim 22, wherein the protein is an antibody.

24. The immobilized substance of claim 23, wherein the thickness of the plasma-polymerized film is 30 to 120 Å.

25. The immobilized substance of claim 21, wherein, to the protein having a binding activity, a second substance modified with a ligand of said protein is bound.

26. The immobilized substance of claim 20, wherein the protein has an enzyme activity.

27. An immobilized polynucleotide comprising the following components:
a) a protein having binding activities, said protein arranged on a surface of a support;
b) a plasma-polymerized film retaining the protein having a binding activity; and
c) a polynucleotide immobilized on the surface of the support through a ligand of the protein having a binding activity.

28. The immobilized polynucleotide of claim 27, wherein polynucleotides having different nucleotide sequences are bound to each of the defined areas on the support.

29. A carrier for immobilizing a polynucleotide, wherein a protein having binding activities is retained on the surface of a support with a plasma-polymerized film.

30. A kit for immobilizing a polynucleotide, said kit comprising the carrier of claim 29 and a primer modified with a ligand of the protein having a binding activity.

31. A method for nucleic acid hybridization assay, said method comprising the following steps of:
a) contacting a sample comprising a nucleic acid to be detected, with the immobilized substance of claim 18 or the immobilized polynucleotide of claim 27; and
b) detecting hybridization of a probe to the nucleic acid in the sample.

32. A method for detecting a ligand, said method comprising the following steps of:
a) contacting a sample comprising a ligand to be detected, with the immobilized substance of claim 17 or 23; and
b) detecting the binding of an antibody or a fragment comprising an antigen binding domain thereof to the ligand in the sample.
